# EUROPÄISCHE PATENTSCHRIFT

(11) **EP 2 757 364 B1**
(45) Veröffentlichungstag und Bekanntmachung des Hinweises auf die Patenterteilung: **26.12.2018**
(21) Anmeldenummer: 13305048.4
(22) Anmeldetag: 17.01.2013
(51) Int. Cl.: G01N 27/04, B82Y 30/00, G01N 33/44, G01R 31/08

(54) **Verwendung einer Polymermischung als Sensormischung**
Use of a polymer mixture as sensing mixture
Utilisation d'un mélange de polymères comme capteur

(43) Veröffentlichungstag der Anmeldung: 23.07.2014
(73) Patentinhaber: Nexans, 92400 Courbevoie (FR)
(72) Erfinder: Dr. Meyer, Matthias, 90419 Nürnberg (DE); Gonnet, Jean-Marc, 90429 Nürnberg (DE); Combessis, Anthony, 13006 Marseille (FR)
(74) Vertreter: Gauer, Pierre

(56) Entgegenhaltungen:
- EP-B1- 1 490 672
- WO-A1-2010/021437
- US-A1- 2010 078 194
- US-A1- 2012 001 128
- MARTNEZ-MORLANES M J ET AL: "Multi-walled carbon nanotubes acting as free radical scavengers in gamma-irradiated ultrahigh molecular weight polyethylene composites", CARBON, ELSEVIER, OXFORD, GB, Bd. 50, Nr. 7, 24. Januar 2012 (2012-01-24), Seiten 2442-2452, XP028470947, ISSN: 0008-6223, DOI: 10.1016/J.CARBON.2012.01.066 [gefunden am 2012-01-31]
- RYAN ET AL: "Carbon nanotubes for reinforcement of plastics? A case study with poly(vinyl alcohol)", COMPOSITES SCIENCE AND TECHNOLOGY, ELSEVIER, UK, Bd. 67, Nr. 7-8, 13. März 2007 (2007-03-13), Seiten 1640-1649, XP005932182, ISSN: 0266-3538, DOI: 10.1016/J.COMPSCITECH.2006.07.006

## Beschreibung

Die Erfindung betrifft eine Polymermischung, die zur Verwendung als Sensormischung geeignet ist.

Eine solche Polymermischung soll zur Bestimmung der mechanischen und/oder elektrischen Stabilität bzw. Alterung eines Kabels eingesetzt werden. Die entsprechende Sensormischung kann sich längs des Kabels erstrecken und einen Anteil des Querschnitts einer extrudierten Schicht des Kabels bilden.

Aus der US 2012/0001128 A1 geht eine für Hochspannungskabel bestimmte, halbleitende Mischung hervor, welche bei 100 Gewichtsteilen eines Basispolyolefins, 0,5 bis 2,15 Gewichtsteile Kohlenstoff-Nanoröhrchen und 0,1 bis 1 Gewichtsteile eines peroxidischen Vernetzungsmittels enthält. Das entsprechende Material soll als den Leiter eines Hochspannungskabels umgebende innere Leitschicht und als dessen Isolierung umgebende äußere Leitschicht eingesetzt werden.

Die EP 1 490 672 B1 beschreibt ein Verfahren zur Bestimmung der Stabilität einer Polymermischung eines Kabels durch Messung des Widerstands bzw. der Leitfähigkeit einer anderen Polymermischung, die 25 Gew-% Ruß oder Metallpartikel als leitfähige Bestandteile enthält. Widerstandsänderungen über die Zeit werden als Maß für die Alterung verwendet, da die Alterung zu einer Verdichtung der leitfähigen Bestandteile durch Schrumpfung führt. Die Polymermischung mit leitfähigen Bestandteilen kann fadenförmig längs des Kabels angeordnet sein, wobei der Widerstand längs der Polymermischung gemessen wird.

Die Aufgabe der Erfindung liegt in der Bereitstellung einer alternativen Polymermischung zur Verwendung als Sensormischung und bevorzugt in der Bereitstellung eines alternativen Verfahrens zur Bestimmung von Änderungen der Polymermischung, bevorzugt in der Bereitstellung alternativer Kabel mit der Polymermischung.

Die Erfindung löst diese Aufgabe entsprechend den Merkmalen des Patentanspruchs 1 durch die Verwendung einer Polymermischung, die zumindest ein Polymer, anorganische Füllstoffe, Verarbeitungshilfsmittel, 0,2 bis 6 Gew.-% Kohlenstoffnanoröhrchen, optional Weichmacher, optional ein Vernetzungssystem und optional Farbstoffe aufweist oder daraus besteht.

Für die Sensormischung sind Anteile der Bestandteile in Gew.-% in Bezug auf die Gesamtmischung angegeben, sofern nicht anders gekennzeichnet.

Das Polymer ist z. B. Polyalkylen, Polypropylen, Polyethylen (PE), bevorzugt PE niedriger Dichte (LDPE), Polyethylenvinylacetat (EVA), Polyurethan und Mischungen dieser und ist z. B. zu bis zu 60 Gew.-% enthalten, bevorzugt zu 20 bis 50 Gew.-%.

Anorganische Füllstoffe sind z. B. Kreide und/oder Kaolin, z. B. zu 5 bis 60 Gew.-% sowie Flammschutzmittel, insbesondere ausgewählt aus Aluminiumtrihydroxid (ATH), z. B. mit einer Teilchengröße von d₅₀ von 1,7 bis 2,1 µm und einer BET-Oberfläche von 3 bis 5 m²/g, z. B. zu 10 bis 60 Gew.-%, bevorzugt zu 40 bis 50 Gew.-%.

Als Alterungsschutzmittel kann z. B. zu 0,3 Gew.-% enthalten sein, z. B. Tetrakis-(methylen-(3,5-di-(tert.)-butyl-4-hydrocinnamat))methan (erhältlich als Irganox 1010).

Das Vernetzungssystem kann z. B. Peroxid und/oder ZnO aufweisen, wobei die Sensormischung nach oder vor Anordnung an oder in einem Kabel vernetzt wird, z. B. durch Erwärmen und/oder Bestrahlen nach ihrer Extrusion. Die Anordnung der Sensormischung kann durch Extrusion als Anteil einer Polymerschicht des Kabels, z. B. als anteiliger Querschnitt des Mantels und/oder einer Innenmantelschicht erfolgen, wobei sich die Sensormischung insbesondere vollständig längs des Kabels erstreckt und ein sich längs des Kabels erstreckendes Filament als Teil des Mantels und/oder der Innenmantelschicht bildet. Alternativ kann die Sensormischung als ein sich längs des Kabels erstreckendes Filament innerhalb des Mantels angeordnet sein.

Die Kohlenstoffnanoröhrchen weisen z.B. einen durchschnittlichen Durchmesser von 8-10 nm, bevorzugt von 9,5 nm auf, insbesondere mit einer durchschnittlichen Länge von 1 bis 5 µm, bevorzugt 1,4 bis 1,6 µm, insbesondere 1,5 µm, bevorzugt mit einem Kohlenstoffanteil von zumindest 90 %, Rest Metalloxide. Kohlenstoffnanoröhrchen weisen z.B. eine BET-Oberfläche von 200 bis 400 g/m² auf, bevorzugt von 250 bis 300 g/m².

Es hat sich gezeigt, dass die Sensormischung durch Extrusion zu Filamenten formbar und anschließend optional vernetzbar ist und eine für die Detektion von alterungsabhängigen Veränderungen ausreichende elektrische Eigenschaften aufweist, insbesondere eine ausreichende Leitfähigkeit. Vorteilhafter Weise reicht ein Gehalt an Kohlenstoffnanoröhrchen in der Sensormischung von 0,2 bis 6 Gew.-%, bevorzugt 0,4 bis 4 oder bis 2 Gew.-% aus, um der Mischung elektrische Eigenschaften zu verleihen, die sich abhängig von der Alterung messbar verändern.

Dieser z.B. Vergleich zum Rußgehalt der EP 1490672 B1 geringe Gehalt an leitfähigen Bestandteilen hat den Vorteil, dass dieser Bestandteil die Eigenschaften gegenüber einer Polymermischung, die bis auf die Kohlenstoffnanoröhrchen identisch ist, nur gering bis unwesentlich verändert, insbesondere in Bezug auf das Alterungsverhalten. Daher ist es bevorzugt, dass die Sensormischung gleich der Polymermischung eines Bestandteils des Kabels, insbesondere gleich der Polymermischung des Mantels oder eines Innenmantels ist, die zusätzlich die Kohlenstoffnanoröhrchen enthält. In dieser Ausführungsform entspricht das Alterungsverhalten der Sensormischung besonders dem Alterungsverhalten der Polymermischung des Mantels oder eines Innenmantels des Kabels, wobei der Gehalt an Kohlenstoffnanoröhrchen, aus dem die Sensormischung mit den Bestandteilen der Polymermischung des Mantels oder eines Innenmantels besteht, der Sensormischugn bzw. dem Filament aus der Sensormischung elektrische Eigenschaften verleiht, deren Änderungen als Maß für die Alterung mit der Vorrichtung messbar sind bzw. im Verfahren gemessen werden.

Die Sensormischung hat im Wesentlichen dieselben bzw. geringfügig veränderte mechanische Eigenschaften wie eine Polymermischung, die mit Ausnahme der Kohlenstoffnanoröhrchen identisch ist. Daher liegt ein Vorteil der Sensormischung bzw. eines Kabels mit zumindest einem Filament aus der Sensormischung darin, dass die mechanischen Eigenschaften des Kabels nicht wesentlich bzw. nicht verschlechtert werden. Dies ist insbesondere für Ausführungsformen von Bedeutung, bei denen ein Filament aus der Sensormischung auf der Außenoberfläche des Mantels oder als Anteil des Querschnitts innerhalb des Mantels oder eines Innenmantels ausgebildet ist.

Das Verfahren zur Bestimmung der Stabilität eines Kabels, das die Sensormischung aufweist, insbesondere in Form eines sich längs des Kabels erstreckenden Filaments aus der Sensormischung, weist die Bestimmung der Änderung zumindest einer elektrischen Eigenschaft der Sensormischung auf, die z.B. die Leitfähigkeit, der Widerstand, die Kapazität und/oder der Dissipationsfaktor ist.

Es hat sich gezeigt, dass die Sensormischung von Einflüssen, die eine Alterung bewirken abhängige Änderungen elektrischer Eigenschaften zeigt, die signifikant sind. Bevorzugt sind die Änderungen elektrischer Eigenschaften der Sensormischung größer als bei einer Polymermischung, die anstelle der erfindungsgemäßen z.B. 0,2 bis 6 Gew.-% Kohlenstoffnanoröhrchen 25% Ruß enthält und ansonsten eine identische Zusammensetzung hat.

In einer ersten Ausführungsform des Kabels ist die Sensormischung längs des Kabels in Form zumindest eines sich längs des Kabels erstreckenden Filaments angeordnet, dessen Enden mit einer Messeinrichtung elektrisch verbunden sind. Bevorzugt ist die Sensormischung längs des Kabels in Form von zwei oder mehr sich längs des Kabels erstreckender Filamente angeordnet, von denen jeweils ein Ende in einem ersten Längsabschnitt des Kabels mit einer Messeinrichtung elektrisch verbunden ist und deren Enden, die in einem von dem ersten Längsabschnitt beabstandeten zweiten Längsabschnitt des Kabels angeordnet sind, elektrisch miteinander verbunden sind. Bevorzugt ist der erste Längsabschnitt ein erstes Ende des Kabels und der zweite Längsabschnitt dessen beabstandetes zweites Ende. Die Filamente können innerhalb des Mantels oder eines Innenmantels angeordnet sein und einen Anteil des Querschnitts des Mantels oder eines Innenmantels ausmachen.

Für diese Ausführungsform kann die Sensormischung z.B. mit der Polymermischung des Mantels bzw. des Innenmantels coextrudiert sein, z.B. mittels einer Düse mit einem Streifenverteiler, dem die Sensormischung zugeführt wird, so dass die Sensormischung einen Streifen in der Polymermischung des Mantels bzw. des Innenmantels bildet.

Alternativ kann die Sensormischung zu einem Filament extrudiert werden, das innerhalb des Mantels angeordnet ist, z.B. dadurch, dass das Filament gemeinsam mit den innerhalb des Mantels anzuordnenden Elementen des Kabels durch eine Extruderdüse geführt wird, mittels derer die Polymermischung des Mantels um diese Elemente des Kabels extrudiert wird. So kann das zumindest eine Filament mit den Adern des Kabels verseilt werden und/oder in einen Zwickel der Adern gelegt werden und mit der Polymermasse eines Innenmantels und/oder Mantels umspritzt werden.

In einer zweiten Ausführungsform bildet die Sensormischung ein Sensorelement, bei dem die Sensormischung zumindest zwei Leiter umfasst. Bevorzugt weist das Sensorelement zumindest zwei Leiter auf, die in einem Abstand innerhalb der Sensormischung angeordnet sind und die Sensormischung kontaktieren, insbesondere über ihre vollständige Länge. Bevorzugt sind die Leiter zueinander parallel innerhalb der Sensormischung angeordnet, wobei die Sensormischung insbesondere als Filament ausgebildet ist, in dem zumindest zwei Leiter voneinander beabstandet und parallel zur Längsachse des Filaments angeordnet sind. In dieser Ausführungsform, in der die Sensormischung mit zumindest zwei beabstandeten Leitern ein Sensorelement bildet, ist die Sensormischung bevorzugt längs des Kabels angeordnet, z.B. auf der Außenfläche des Kabelmantels und/oder innerhalb des Kabelmantels. Bei Anordnung des Sensorelements innerhalb des Kabelmantels kann dieses gemeinsam mit den weiteren Elementen des Kabels, die vom Mantel umfasst sind, angeordnet werden und mit der Polymermischung des Mantels umspritzt werden. Das Sensorelement kann, wie auch mit Bezug auf die erste Ausführungsform beschrieben, mit den Adern verseilt und/oder in deren Zwickel gelegt sein.

In der zweiten Ausführungsform ist zur Messung jeder innerhalb der Sensormischung angeordneter Leiter des Sensorelements mit einer Messeinrichtung verbunden. In dieser Ausführungsform ist die Messeinrichtung bevorzugt zur Messung bzw. Bestimmung der Kapazität und/oder des Dissipationsfaktors eingerichtet.

Es hat sich gezeigt, dass die elektrischen Eigenschaften der Sensormischung stark durch Temperaturerhöhungen beeinflusst werden. So kann z.B. die Leitfähigkeit der Sensormischung durch Temperaturerhöhungen schneller bzw. stärker erhöht werden, als dies durch die Alterung bewirkt wird, die z.B. eine Schrumpfung und/oder Versprödung verursacht.

Entsprechend weist ein Kabel zusätzlich zu der Sensormischung bevorzugt einen Temperatursensor auf und weiter bevorzugt eine Auswerteeinrichtung, die eingerichtet ist, Temperaturmesswerte zu speichern und mit den Messwerten für elektrische Eigenschaften der Sensormischung zu korrelieren. Weiter bevorzugt weist eine solche Auswerteeinrichtung Daten für eine zuvor für dieselbe Sensormischung bestimmte Korrelation zwischen Temperaturmesswerten und elektrischen Eigenschaften auf und ist eingerichtet, die die zuvor bestimmte Korrelation bei der Auswertung der Messwerte für die elektrische Eigenschaft der Sensormischung abhängig von den Temperaturmesswerten einzubeziehen. Durch das Einbeziehen der zuvor bestimmten Korrelation der Temperaturmesswerte bei der Auswertung der Messwerte für die elektrische Eigenschaft der Sensormischung wird die Genauigkeit der Bestimmung der Alterung durch Einflüsse, die nicht durch Temperaturerhöhungen verursacht werden, erhöht.

Ein bevorzugter Temperatursensor ist eine optische Faser, z.B. eine Glas- oder Lichtleitfaser, die längs innerhalb des Kabels enthalten ist. Bevorzugt ist eine optische Faser angrenzend an die Sensormischung angeordnet. Z.B. kann die optische Faser in der zweiten Ausführungsform der Sensormischung als Sensorelement innerhalb der Sensormischung angeordnet sein, z.B. parallel zu den Leitern des Sensorelements. Eine solche optische Faser ist z.B. mit einem optischen Detektor verbunden, der zur Messung der optischen Eigenschaften eingerichtet ist, insbesondere zur Messung der Raman-Streuung, die z.B. durch Einstrahlen von Laserstrahlung in die optische Faser erzeugt wird. Entsprechend kann die optische Faser mit einem Laser zur Einstrahlung von Laserstrahlung in die Faser versehen sein und mit einem optischen Detektor, der insbesondere an dem Ende der optischen Faser angeordnet ist, in das die Laserstrahlung eingestrahlt wird. Bevorzugt ist eine optische Faser, deren optische Eigenschaften nur temperaturabhängig sind. Z.B. kann eine optische Faser verwendet werden, die eine hohe Beständigkeit gegen radioaktive Strahlung aufweist, z.B. mit hohem OH-Gehalt, mit Fluor- und/oder Bromdotierung.Ein Vorteil eines erfindungsgemäßen Kabels, das zusätzlich zu der Sensormischung eine optische Faser aufweist, liegt darin, dass die optische Faser bei radioaktiver Bestrahlung eine Änderung ihrer optischen Eigenschaften erfährt, z.B. eine Trübung und/oder Dämpfung. Daher können beim Verfahren die optischen Messwerte zur Bestimmung eines Anteils der Alterung herangezogen werden, der durch radioaktive Strahlung auf das Kabel verursacht wird. Entsprechend kann die Auswerteeinrichtung eingerichtet sein, die optischen Messwerte, die eine radioaktive Bestrahlung des Kabels anzeigen, zur Bestimmung der Alterung des Kabels heranzuziehen. Eine solche optische Faser weist bevorzugt eine Glaszusammensetzung auf, deren optische Eigenschaften durch radioaktive Bestrahlung, die z.B. γ-Strahlung ist, veränderlich sind, insbesondere temperaturunabhängig. Solche optischen Fasern sind z.B. solche mit niedrigem Gehalt an Fluor und/oder an Brom, solche aus reinem Siliziumdioxid, und/oder solche mit niedrigem OH-Gehalt. Entsprechend kann das Kabel eine optische Faser aufweisen, deren optische Eigenschaften temperaturabhängig sind und/oder eine optische Faser, deren optische Eigenschaften von radioaktiver Strahlung veränderlich sind. Diese Eigenschaften, z.B. eine Trübung, können als Dämpfung in dB/km bestimmt werden.

Bevorzugt ist der Detektor jeder optischen Faser mit einer Auswerteeinrichtung gekoppelt, die vorbestimmte Messdaten gespeichert hat, die die Korrelation der Messwerte für die Temperatur und/oder für radioaktive Strahlung mit der Alterung des Kabels bzw. zumindest einer Polymermischung des Kabels, insbesondere Mantelmischung und/oder der Sensormischung, enthalten.

### Genaue Beschreibung der Erfindung

Die Erfindung wird nun genauer anhand von beispielhaften Sensormischungen und mit Bezug auf die Figuren beschrieben, die schematisch
- in Figur 1 ein beispielhaftes Sensorelement,
- in Figur 2 eine Ausführung eines Kabelmantels
- in Figur 3 einen Querschnitt durch eine Ausführungsform eines Kabelmantels mit Sensorelement und
- in Figur 4 einen Querschnitt durch eine weitere Ausführungsform eines Kabelmantels mit Sensorelement zeigen.
Als einfache Ausführungsform der Sensormischung wurde Kohlenstoffnanoröhrchen (durchschnittlicher Durchmesser ca. 9,5 nm, durchschnittliche Länge ca. 1,5 µm, 90% Kohlenstoff, 10% Metalloxide, BET-Oberfläche 250-300 g/m²) in Mischung 1) zu 2,1 oder in Mischung 2) zu 6,5 Gew.-% in 97,9 Gew.-% bzw. 93,5 Gew.-% LDPE gemischt und durch Extrusion geformt.

Die Messung des elektrischen Widerstands für Mischung 1) ergab die folgenden Werte bei Alterung bei 100 °C:

| | den elektrischen Widerstand (Ohm) | die Reißdehnung (%) |
|---|---|---|
| am Tag 0 | 1,52 x 10¹¹ | 143 |
| Tag 7 | 7,34 x 10¹⁰ | nicht bestimmt |
| Tag 45 | 8,55 x 10⁸ | 51 |

Für Mischung 1) konnte eine von der Alterung abhängige Änderung des Widerstands gemessen werden. Die Alterung wurde stellvertretend für die Festigkeit als Abnahme der Reißdehnung bestimmt.

Für Mischung 2) konnte keine Änderung des Widerstands mit der Alterung festgestellt werden. Dies wird darauf zurückgeführt, dass der Gehalt an Kohlenstoffnanoröhrchen zu hoch war, um alterungsbedingte Änderungen des elektrischen Widerstands anzuzeigen.

Die folgenden Sensormischungen zeigen eine messbare Änderung der elektrischen Eigenschaften in Abhängigkeit von einer Alterung:

| | Mischung | | | | | | | | | | |
|---|---|---|---|---|---|---|---|---|---|---|---|
| Inhaltsstoff | 3) | 4) | 5) | 6) | 7) | 8) | 9) | 10) | 11) | 12) | 13) |
| KNR | 1,8 | 1,8 | 0,2 | 0,7 | 1,0 | 1,6 | 2,3 | 3 | 4 | 1,8 | 1,8 |
| LDPE | 22 | - | - | 22 | 22 | 22 | 22 | 22 | 22 | 48 | 48 |
| EVA | 26 | 48 | 49,6 | 27,1 | 26,8 | 26,2 | 25,5 | 24,8 | 23,8 | - | 44 |
| ATH | 44 | 44 | 44 | 44 | 44 | 44 | 44 | 44 | 44 | 44 | - |
| ASM | 1 | 1 | 1 | 1 | 1 | 1 | 1 | 1 | 1 | 1 | 1 |
| Peroxid | 0,1 | 0,1 | 0,1 | 0,1 | 0,1 | 0,1 | 0,1 | 0,1 | 0,1 | 0,1 | 0,1 |
| Verarbeitungshilfsmittel | 5,1 | 5,1 | 5,1 | 5,1 | 5,1 | 5,1 | 5,1 | 5,1 | 5,1 | 5,1 | 5,1 |

| | | | | | | | | | | | |
|---|---|---|---|---|---|---|---|---|---|---|---|
| KNR = Kohlenstoffnanoröhrchen, ASM = Alterungsschutzmittel | | | | | | | | | | | |

Am Beispiel von Mischung 3) kann gezeigt werden, dass der Gehalt an Kohlenstoffnanoröhrchen die mechanischen Eigenschaften nicht verschlechtert. Nachfolgend sind die mechanischen Eigenschaften von Mischung 3) im Vergleich mit Mischung V3) gezeigt, die ohne den Gehalt an Kohlenstoffnanoröhrchen ansonsten dieselbe Zusammensetzung aufwies:

| Eigenschaft | Mischung 3) | Mischung V3) |
|---|---|---|
| Reißdehnung (%) | 123 | 160 |
| Zugfestigkeit (MPa) | 12,8 | 11 |
| Dehnung nach 7 Tagen bei 100°C (%) | 104 | - |
| Zugfestigkeit nach 7 Tagen bei 100°C (MPa) | 13,7 | - |

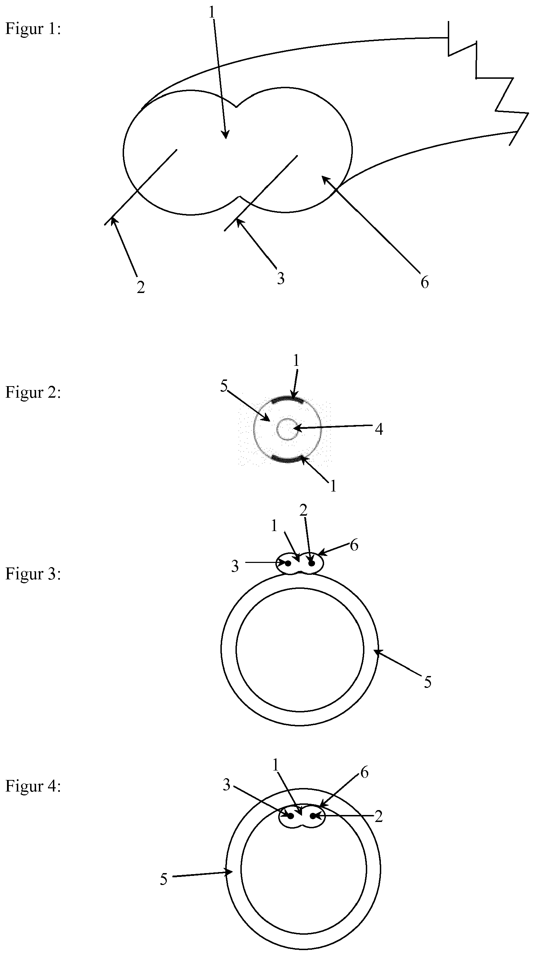

Die Figur 1 zeigt ein Sensorelement 6 aus einer Sensormischung 1, in der zwei voneinander beabstandete Leiter 2, 3 jeweils parallel zur Längsachse der extrudierten Sensormischung angeordnet sind. Die Enden der Leiter 2, 3 sind jeweils mit einer Messeinrichtung zu kontaktieren.

In einem Test wurde für ein Sensorelement von insgesamt 5m Länge nach Figur 1 die Änderung des elektrischen Widerstands gemessen. In diesem Test wurde gefunden, dass der Widerstand mit zunehmender Alterung durch Temperaturbelastung (Ofenalterung) zunahm. Die Sensormischung wies die Zusammensetzung 3) auf und enthielt 1.8 Gew.-% Kohlenstoffnanoröhrchen.

Der Widerstand wurde mit einem Handmessgerät zwischen den beiden Leitern 2, 3 bestimmt. Vor der Alterung betrug der Widerstand 167,6 Ohm, nach Ofenalterung bei 100°C für 7d 819 Ohm, nach 100°C für 40d 11.160 Ohm. In einem entsprechenden Test mit ursprünglich identischem Sensorelement wurde der Widerstand nach Ofenalterung bei 80°C für 7d zu 658 Ohm, bei 80°C für 40d zu 1.608 Ohm gemessen.

Der Widerstand der beiden Leiter 2, 3 selbst hatte sich durch die Ofenalterung nicht wesentlich geändert, so dass in diesem Test die Sensormischung eine Zunahme des Widerstands durch Alterung zeigte.

Die Figur 2 zeigt schematisch den Querschnitt durch ein Kabel, das zwei beabstandete Filaments aus einer Sensormischung aufweist, die als Anteil des Querschnitts des Mantels 5 extrudiert sind. Dies entspricht der ersten Ausführungsform, bei der ein Filament bzw. Enden von miteinander verbundenen Filamenten aus Sensormischung mit einer Messeinrichtung zur verbinden sind.

In den Figuren 3 und 4 sind weder Adern noch Innenmantel des Kabels dargestellt.

Die Figur 3 zeigt ein Kabel, bei dem ein Sensorelement 6 auf der Außenfläche des Mantels 5 angeordnet ist.

Figur 4 zeigt ein Kabel, bei dem ein Sensorelement 6 innerhalb des Mantels 5 angeordnet ist, z.B. dadurch hergestellt, dass beim Verfahren zur Herstellung die vom Mantel umfassten Elemente des Kabels einschließlich des Sensorelements 6 von der Polymermischung umspritzt werden, die den Mantel bildet.

### Bezugszeichenliste:

- 1: Sensormischung
- 2: Leiter
- 3: Leiter
- 4: Ader
- 5: Mantel
- 6: Sensorelement

## Patentansprüche

1. Verwendung einer Polymermischung als Sensormischung (1) zur Bestimmung der Alterung eines Kabels, wobei die Polymermischung zumindest ein Polymer, anorganische Füllstoffe, Verarbeitungshilfsmittel, 0,2 bis 6 Gew.-% Kohlenstoffnanoröhrchen, optional Weichmacher, optional ein Vernetzungssystem und optional Farbstoff aufweist oder daraus besteht.

2. Verwendung nach Anspruch 1, **dadurch gekennzeichnet, dass** die Kohlenstoffnanoröhrchen einen durchschnittlichen Durchmesser von 8-10 nm, eine durchschnittliche Länge von 1 bis 5 µm, einen Kohlenstoffanteil von zumindest 90 %, Rest Metalloxide und eine BET-Oberflache von 200 bis400 g/m² aufweisen.

3. Verwendung nach Anspruch 1 oder 2, **dadurch gekennzeichnet, dass** die Polymermischung bis 60 Gew.-% Flammschutzmittel enthält.

4. Kabel mit einem Mantel (5), der zumindest eine Ader umfaßt, und mit einer Polymermischung nach Anspruch 1, **dadurch gekennzeichnet, dass** die Sensormischung (1) ein Sensorelement (6) bildet, das um zumindest zwei voneinander beabstandete Leiter (2,3) herum angeordnet ist.

5. Kabel mit einem Mantel (5), der zumindest eine Ader umfaßt, welches ein Filament aus einer Polymermischung nach Anspruch 1 aufweist, wobei das Filament in dem Mantel angeordnet ist und sich entlang der vollständigen Länge des Kabels erstreckt und wobei die Enden des Filaments dafür ausgebildet sind, mit einer Messeinrichtung elektrisch verbunden zu werden.

6. Kabel nach Anspruch 4 oder 5, **dadurch gekennzeichnet, dass** zumindest eine optische Faser längs des Kabels angeordnet ist, die mit einem optischen Detektor verbunden ist, dessen Messwerte einer mit einer Messeinrichtung verbundenen Auswerteeinrichtung zugeführt werden, die eingerichtet ist, Messwerte der Messeinrichtung mit den Messwerten des optischen Detektors zu korrelieren, wobei zumindest eine optische Faser eine Glaszusammensetzung aufweist, deren optische Eigenschaften temperaturabhängig sind und/oder zumindest eine optische Faser eine Glaszusammensetzung aufweist, deren optische Eigenschaften durch radioaktive Bestrahlung veränderlich sind.

7. Verfahren zur Bestimmung der Alterung eines Kabels nach einem der Ansprüche 4 bis 6, **dadurch gekennzeichnet, dass** Messwerte für die Leitfähigkeit, den Widerstand, die Kapazität und/oder den Dissipationsfaktor der Sensormischung (1) bestimmt werden.

8. Verfahren nach Anspruch 7, **dadurch gekennzeichnet, dass** längs des Kabels eine optische Faser angeordnet wird, deren optische Eigenschaften temperaturabhängig sind, wobei Messwerte für deren temperaturabhängige optische Eigenschaften gemessen werden und/oder dass das Kabel eine längs des Kabels angeordnete optische Faser enthalt, deren optische Eigenschaften von radioaktiver Bestrahlung abhängig sind, wobei Messwerte für deren von radioaktiver Bestrahlung abhängige optische Eigenschaften gemessen werden.

9. Verfahren nach Anspruch 7 oder 8, **dadurch gekennzeichnet, dass** die Messwerte für die Leitfähigkeit, den Widerstand, die Kapazität und/oder den Dissipationsfaktor der Sensormischung (1), Messwerte für temperaturabhängige optische Eigenschaften und/oder Messwerte für von radioaktiver Bestrahlung abhängige optische Eigenschaften in einer Auswerteeinrichtung mit Daten in Bezug gesetzt werden, die eine vorbestimmte Korrelation der Alterung zumindest einer Polymermischung des Kabels mit diesem Messwert enthalten.

10. Verfahren nach einem der Ansprüche 6 bis 9, **dadurch gekennzeichnet, dass** die Sensormischung (1) in ein Sensorelement (6) geformt wird, das zumindest zwei voneinander beabstandete Leiter (2,3) enthält und das Sensorelement (6) gemeinsam mit weiteren Elementen des Kabels mit einer Polymermischung für dessen Mantel umspritzt wird.

## Claims

1. A use of a polymer mixture as sensor mixture (1) for determining the aging of a cable, wherein the polymer mixture has or consists of at least one polymer, inorganic fillers, processing additives, 0.2 to 6% by weight carbon nanotubes, optionally plasticizer, optionally a crosslinking system and optionally a coloring agent.

2. A use according to Claim 1, **characterized in that** the carbon nanotubes have an average diameter of 8-10 nm, an average length of 1 to 5 µm, a carbon content of at least 90%, the rest metal oxides and a BET surface area of 200 to 400 g/m².

3. A use according to Claim 1 or 2, **characterized in that** the polymer mixture contains up to 60% by weight of flame retardant.

4. A cable with a sheath (5), which comprises at least one wire, and with a polymer mixture according to Claim 1, **characterized in that** the sensor mixture (1) forms a sensor element (6), which is arranged around at least two conductors (2, 3) spaced apart from one another.

5. A cable with a sheath (5), which comprises at least one wire, which has a filament made of a polymer mixture according to Claim 1, wherein the filament is arranged in the sheath and extends along the complete length of the cable and wherein the ends of the filament are designed to be connected electrically to a measuring device.

6. A cable according to Claim 4 or 5, **characterized in that** at least one optical fiber is arranged along the cable, which is connected to an optical detector, the measured values of which are supplied to an evaluation device connected to a measuring device, which is set up to correct measured values of the measuring device with the measured values of the optical detector, wherein at least one optical fiber has a glass composition, the optical properties of which are temperature-dependent and/or at least one optical fiber has a glass composition, the optical properties of which can be varied by radioactive irradiation.

7. A method for determining the aging of a cable according to any one of Claims 4 to 6, **characterized in that** measured values are determined for the conductivity, the resistance, the capacitance and/or the dissipation factor of the sensor mixture (1).

8. A method according to Claim 7, **characterized in that** along the cable an optical fiber is arranged, the optical properties of which are temperature-dependent, wherein measured values are measured for its temperature-dependent optical properties and/or that the cable contains an optical fiber arranged along the cable, the optical properties of which are dependent on radioactive irradiation, wherein measured values are measured for their optical properties dependent on radioactive irradiation.

9. A method according to Claim 7 or 8, **characterized in that** the measured values for the conductivity, the resistance, the capacitance and/or the dissipation factor of the sensor mixture (1), measured values for temperature-dependent optical properties and/or measured values for optical properties dependent on radioactive irradiation are related in an evaluation device to data, which contain a predetermined correlation of the aging at least of one polymer mixture of the cable with said measured value.

10. A method according to any one of Claims 6 to 9, **characterized in that** the sensor mixture (1) is formed into a sensor element (6), which contains at least two conductors (2, 3) spaced apart from one another and the sensor element (6) together with further elements of the cable is injection-molded around with a polymer mixture for its sheath.

## Revendications

1. Utilisation d'un mélange de polymères sous la forme d'un mélange capteur (1) pour la détermination du vieillissement d'un câble, le mélange de polymères comprenant ou étant constitué d'au moins un polymère, de charges inorganiques, de moyens auxiliaires de traitement, de 0,2 à 6 % en poids de nanotubes de carbone, en option des plastifiants, en option un système de réticulation et en option un colorant.

2. Utilisation selon la revendication 1, **caractérisé en ce que** les nanotubes de carbone présentent un diamètre moyen de 8 à 10 nm, une longueur moyenne de 1 à 5 µm, une part de carbone d'au moins 90 %, le reste d'oxydes métalliques et une surface BET de 200 à 400 g/m².

3. Utilisation selon la revendication 1 ou 2, **caractérisé en ce que** le mélange de polymères contient jusqu'à 60 % en poids de produits de protection contre les flammes.

4. Câble avec une enveloppe externe (5) qui comprend au moins un fil et avec un mélange de polymères selon la revendication 1, **caractérisé en ce que** le mélange capteur (1) constitue un élément de capteur (6) qui est disposé autour d'au moins deux conducteurs (2, 3) distants entre eux.

5. Câble avec une enveloppe externe (5) qui comprend au moins un fil, qui comprend un filament constitué d'un mélange de polymères selon la revendication 1, le filament étant disposé dans l'enveloppe externe et s'étendant le long de toute la longueur du câble et les extrémités du filament étant conçues pour être reliées électriquement avec un dispositif de mesure.

6. Câble selon la revendication 4 ou 5, **caractérisé en ce qu'**au moins une fibre optique est disposée le long du câble, qui est reliée avec un détecteur optique, dont les valeurs de mesure sont introduites dans un dispositif d'analyse, relié avec un dispositif de mesure, qui est conçu pour corréler les valeurs de mesure du dispositif de mesure avec les valeurs de mesure du détecteur optique, au moins une fibre optique présentant une composition de verre dont les propriétés optiques dépendent de la température et/ou au moins une fibre optique présente une composition de verre dont les propriétés optiques peuvent être modifiées par une irradiation radioactive.

7. Procédé de détermination du vieillissement d'un câble selon l'une des revendications 4 à 6, **caractérisé en ce que** les valeurs de mesure pour la conductivité, la résistance, la capacité et/ou le facteur de dissipation du mélange capteur (1) sont déterminées.

8. Procédé selon la revendication 7, **caractérisé en ce que**, le long du câble, une fibre optique est disposée, dont les propriétés optiques dépendent de la température, des valeurs de mesure pour ses propriétés optiques dépendant de la température étant mesurées et/ou **en ce que** le câble contient une fibre optique disposée le long du câble, dont les propriétés optiques dépendent d'une irradiation radioactive, des valeurs de mesure pour ses propriétés optiques dépendant d'une irradiation radioactive étant mesurées.

9. Procédé selon la revendication 7 ou 8, **caractérisé en ce que** les valeurs de mesure pour la conductivité, la résistance, la capacité et/ou le facteur de dissipation du mélange capteur (1), des valeurs de mesure pour ses propriétés optiques dépendant de la température et des valeurs de mesure pour ses propriétés optiques dépendant d'une irradiation radioactive étant comparées dans un dispositif d'analyse avec des données, qui contiennent une corrélation prédéterminée du vieillissement d'au moins un mélange de polymères du câble avec cette valeur de mesure.

10. Procédé selon l'une des revendications 6 à 9, **caractérisé en ce que** le mélange capteur (1) est moulé en un élément capteur (6) qui contient au moins deux conducteurs (2, 3) distants entre eux et l'élément capteur (6) est surmoulé, conjointement avec d'autres éléments du câble, avec un mélange de polymères pour son enveloppe externe.
